(19) Europäisches Patentamt
European Patent Office
Office européen des brevets

(11) Publication number: **0 133 376**
**B1**

(12) # EUROPEAN PATENT SPECIFICATION

(45) Date of publication of patent specification: **27.07.88**

(51) Int. Cl.$^4$: **C 07 D 307/58,**
C 07 D 407/04, A 61 K 31/365

(21) Application number: **84305301.8**

(22) Date of filing: **03.08.84**

(54) **Synthetic analgesic and/or anti-inflammatory derivatives of manoalide.**

(30) Priority: **03.08.83 US 519852**
**03.08.83 US 519853**
**18.06.84 US 621879**

(43) Date of publication of application:
**20.02.85 Bulletin 85/08**

(45) Publication of the grant of the patent:
**27.07.88 Bulletin 88/30**

(84) Designated Contracting States:
**DE FR GB IT**

(56) References cited:
**AT-B- 362 873**

**CHEMICAL ABSTRACTS, vol. 87, no. 3, July 18, 1977, Columbus, Ohio, USA F. BOHLMANN et al. "Naturally occuring terpene derivatives, 82. Constituentes from representatives of the Eupatorium group" page 306, columns 1,2; abstract-no. 18 971Y**

**CHEMICAL ABSTRACTS, vol. 93, no. 17, October 27, 1980, Columbus, Ohio, USA E. D. DE SILVA et al.: "Manoalide, an antibiotic sesterterpenoid from the marine sponge Luffariella variabilis (Polejaeff)" page 697, column 2, abstract-no. 168 442q**

(73) Proprietor: **THE REGENTS OF THE UNIVERSITY OF CALIFORNIA**
**2199 Addison Street**
**Berkeley California 94720 (US)**

(72) Inventor: **Jacobs, Robert S.**
**1431 Portesuello**
**Santa Barbara California 93105 (US)**
Inventor: **Faulkner, John D.**
**5915 Desert View Drive**
**La Jolla California 92037 (US)**

(74) Representative: **Woods, Geoffrey Corlett et al**
**J. A. KEMP & CO.**
**14 South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 133 376 B1

Courier Press, Leamington Spa, England.

**Description**

The present invention relates to synthetic analogs of the marine natural product manoalide, their preparation and pharmaceutical compositions containing them.

Manoalide is a marine natural product derived from the marine sponge *Luffariella variabilis*, as reported by E. D. de Silva and P. J. Scheuer, Tetrahedron Letters, volume 21, pages 1611—1614, Pergamon Press Ltd, 1980. Manoalide is disclosed as having analgesic and/or anti-inflammatory activity in US—A—4447445. US—A—4447445 was published May 8, 1984 and corresponds to U.S. Application No. 519852, which is one of the applications from which the present case claims priority.

The invention provides manoalide derivatives of general formula (I):

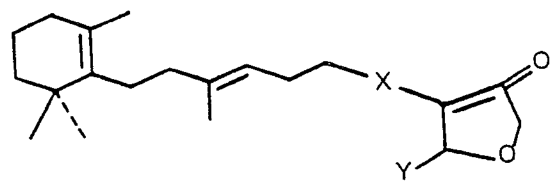

(I)

wherein X is

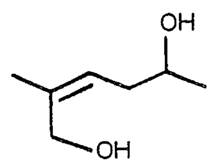

and Y is a hydrogen atom, or X is

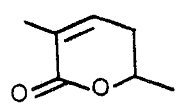

and Y is a hydroxyl or acetoxy group, or X is

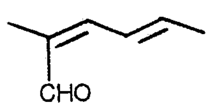

and Y is a hydroxyl group.

Compounds of the invention are 3,7-bis(hydroxymethyl)-4-hydroxy-11-methyl-13-(2′,6′,6′-trimethylcyclohexenyl)-2,6,10-tridecatrienoic acid δ-lactone, manoalide δ-lactone, manoalide δ-lactone acetate and dehydromanoalide.

The invention further provides for the preparation of 3,7-bis(hydroxymethyl)-4-hydroxy-11-methyl-13-(2′,6′,6′-trimethylcyclohexenyl)-2,6,10-tridecatrienoic acid δ-lactone, which process comprises reacting manoalide with excess sodium borohydride.

Additionally, the invention provides a process for the preparation of manoalide δ-lactone or manoalide δ-lactone acetate, which process comprises reacting manoalide with Jones' reagent and, if desired, reacting the manoalide δ-lactone thus produced with acetic anhydride to form manoalide δ-lactone acetate.

The invention also provides a process for the preparation of dehydromanoalide, which process comprises the acid-catalysed dehydration of manoalide.

The present synthetic analogs of manoalide have anti-inflammatory activity greater than that of indomethacin and less than that of hydrocortisone. Potential uses include treatment of rheumatoid arthritis, osteoarthritis, rheumatic carditis, collagen and/or auto-immune diseases such as myasthenia gravis, allergic diseases, bronchial asthma and ocular and skin inflammatory diseases. The compounds of the invention have analgesic activity and are also likely to be useful as adjuvant therapy associated with organ and tissue transplants and as a local treatment for any venom in which a major constituent is the enzyme phospholipase $A_2$. Since manoalide blocks oxazolone induced inflammation these compounds would be useful in treating forms of allergic contact dermatitis (such as poison oak or poison ivy).

The synthetic analogs of manoalide of the invention may be administered to mammals including humans in an effective amount of 10 to 50 mg per kilogram of body weight. The compounds may be administered orally, parenterally or by other standard administration routes. The dosage form may be by tablet containing normal acceptable additives, excipients, etc. The parenteral form contains typical aqueous intravenous solution ingredients.

The invention therefore also provides pharmaceutical compositions comprising a manoalide derivative of general formula (I) as active ingredient, together with a pharmaceutically acceptable additive or excipient.

Yet further, the present invention provides the use of manoalide in the preparation of a pharmaceutical composition for use as an analgesic and/or anti-inflammatory agent.

The following Examples illustrate the invention. In the Examples, parts and percentages are by weight unless otherwise indicated.

Example I

Preparation of 3,7-Bis(hydroxymethyl)-4-hydroxy-11-methyl-13-(2′,6′,6′-trimethylcyclohexenyl)-2,6,10-tridecatrienoic acid γ-lactone.

Excess sodium borohydride (300 mg, 7.0 mmole) was added in small portions to a stirred solution of manoalide (136 mg, 0.33 mmole) in isopropanol (20 mL) at 0°C. The mixture was stirred at 0°C for one hour. Excess reagent was destroyed by dropwise addition of 2% hydrochloric acid until hydrogen evolution ceased. The product was partitioned between water (100 mL) and ether (2 × 100 mL), the ether extract dried over sodium sulfate and the solvent removed to obtain an oil. The product was purified by HPLC to obtain the diol. Yield 75 mg (55% theoretical); oil;* $^1$H NMR ($CDCl_3$) δ 0.99 (s, 6 H), 1.60 (s, 3 H), 1.65 (s, 3 H), 4.11 (d, 1 H, $J$ = 14 HZ), 4.17 (d, 1 H, $J$ = 14 Hz), 5.39 (t, 1 H, $J$ = 7 Hz), 5.98 (br s, 1 H); HRMS. $m/z$ 402.2770, $C_{25}H_{38}O_4$ requires 402.2770.

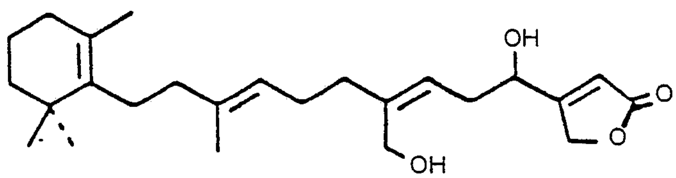

*IR (film) 3350, 1775 cm$^{-1}$

Example II

Preparation of manoalide δ-lactone.

A solution of Jones' reagent [prepared from chromium trioxide (6.7 g) and sulfuric acid (g mL)] was added dropwise to a stirred solution of manoalide (30 mg, 0.07 mmole) in distilled acetone (20 mL) at 25°C until the solution remained brown. After five minutes, the reaction mixture was filtered through a short column of silica gel and the solvent evaporated to obtain an oil. The product was chromatographed by HPLC to obtain the manoalide δ-lactone as a mixture of two diastereoisomers. Yield 15 mg (50% theoretical); oil;* $^1$H NMR ($CDCl_3$) δ 0.99 (s, 6 H), 1.60 (s, 3 H), 1.65 (s, 3 H), 5.10 (m, 1 H), 5.26 (dd, 0.5 H, $J$ = 12, 5 Hz), 5.37 (dd, 0.5 H, $J$ = 12, 5 Hz), 6.15 (s, 0.5 H), 6.20 (d, 0.5 H, $J$ = 7 Hz), 6.23 (s, 0.5 H), 6.35 (d, 0.5 H, $J$ = 7 Hz), 6.62 (m 0.5 H), 6.65 (m, 0.5 H); HRMS. $m/z$ 414.2384, $C_{25}H_{34}O_5$ requires 414.2406.

Manoalide δ-lactone is an inseparable 1:1 mixture of diastereoisomers resulting from epimerization at the hemiacetal carbon atom.

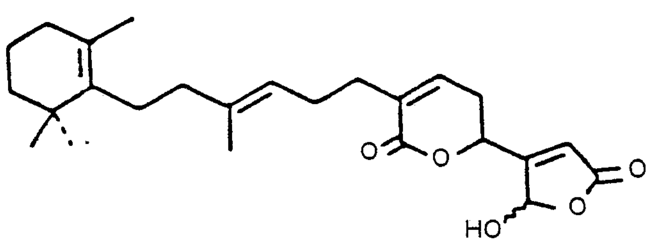

*IR (film) 3300, 1770, 1740 cm$^{-1}$; UV(MeOH) 208.5 nm (ε 10,350)

Example III

Preparation of manoalide δ-lactone acetate.

Manoalide δ-lactone (15 mg, 0.04 mmole) was dissolved in acetic anhydride (0.5 mL) and pyridine (1.0 mL) and the mixture was stirred at 25°C for four hours. The solvents were removed under high vacuum and the residue dissolved in either and filtered through a silica gel plug to obtain a clear oil. The oil was chromatographed by HPLC to obtain a mixture of diastereoisomeric acetates. Yield 16 mg (quantitative; oil;* $^1$H NMR ($CDCl_3$) δ 0.99 (s, 3 H), 1.59 (s, 3 H), 1.65 (s, 3 H), 2.18 (s, 3 H), 5.10 (t, 1 H, $J$ = 7 Hz), 5.21 (m, 1 H), 6.26 (s, 0.4 H), 6.34 (s, 0.6 H), 6.61 (m, 1 H), 6.98 (s, 1 H) HRMS. $m/z$ 456.2514, $C_{27}H_{36}O_6$ requires 456.2512.

Manoalide δ-lactone acetate is a 6:4 mixture of two diastereoisomers. The diastereoisomers can be separated but the material assayed was the mixture of isomers.

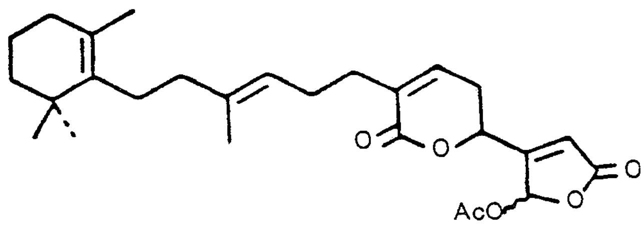

*IR (film) 1800, 1770, 1725 $cm^{-1}$; UV(MeOH) 208 nm (ε 10,600)

Example IV

Mouse Ear Anti-inflammatory Assay

Test compound and phorbol myristate acetate (PMA) are topically applied simultaneously to the left ears of mice. Three hours, 20 minutes after application, the mice are sacrificed, left and right ears removed and standard sized bores taken. Edema (inflammation) is measured as the difference in weight between left and right ears [Van Arman, C. G., Clin. Pharmacol. Ther. 16:900—904 (1974)].

TABLE I
Inhibition of PMA-induced inflammation**
by manoalide and manoalide derivatives

| Compound | Dose (µg/ear) | Average ear weight (mg/ear) |
|---|---|---|
| Manoalide | 7.5 | 20.45 |
| | 10.0 | 19.75 |
| | 20.0 | 17.00* |
| | 40.0 | 16.80* |
| | 80.0 | 12.90* |
| | 160.0 | 12.60* |
| | 320.0 | 11.20* |
| Manoalide diol | 25.0 | 14.54* |
| | 50.0 | 13.17* |
| Manoalide δ-lactone | 25.0 | 16.38* |
| | 50.0 | 14.87* |
| Manoalide δ-lactone acetate | 25.0 | 16.64* |
| | 50.0 | 13.34* |

* Statistically significant difference, student's T-test, P ⩽ .05
** PMA dose = 1.5 µg/ear

RESULTS

As shown in Table I, the level of activity of all three compounds is approximately equivalent to that of manoalide when tested for the ability to inhibit PMA-induced inflammation in the mouse ear. For purposes of comparison, the average weight of an untreated mouse ear is 9.3 mg (100% inhibition) and the average weight of an ear treated with PMA alone is 23 mg (0% inhibition). Manoalide acetate was compared with manoalide in a separate study using a higher concentration of PMA and was also found to be active (Table II).

TABLE II
Inhibition of PMA-induced inflammation**
by manoalide and manoalide derivatives

| Compound | Dose (µg/ear) | Average ear weight (mg/ear) |
|---|---|---|
| Manoalide | 300.0 | 17.46* |
| Manoalide acetate | 300.0 | 19.01* |
| | 150.0 | 21.53 |

* Statistically significant difference, student's T-test, P = .05
** PMA dose = 5.0 µg/ear

Example V
*Isolation and Characterization of dehydromanoalide*

Dehydromanoalide is a synthetic derivative of manoalide that has not been reported to date. Examination of UV and $^{1}H$ NMR data of the crude extracts of the sponge *Luffariella variabilis* provided evidence that dehydromanoalide was *not* a natural product but was formed during chromatography, presumably by acid-catalyzed dehydration of manoalide on silica.

The isolation and purification of manoalide requires two or three chromatographic separations on silica gel. Fractions that eluted before manoalide were saved and certain fractions, distinguished by their $^{1}H$ NMR spectra, combined. The combined fractions were chromatographed by LC on µ-Porasil using diethyl ether as eluant to obtain dehydromanoalide as a viscous yellow oil. As is typical of an artifact of isolation, the yield is variable.

*Dehydromanoalide*
UV (EtOH) 316 nm (ε 12000), 205 nm (ε 10300)
UV (EtOH+NaOH) 461 nm (ε 25000), 280 nm (ε 1600), 246 (ε 2000)
IR ($CHCl_3$) 1745 $cm^{-1}$, 1670 $cm^{-1}$
$^{1}H$ NMR ($CDCl_3$) δ 0.96 (s, 6 H), 1.56 (s, 3 H), 1.60 (s, 3 H), 5.11 (br t, 1 H, $J$ = 7 Hz), 6.14 (s, 1 H), 6.32 (s, 1 H), 6.82 (d, 1 H, $J$ = 15.5 Hz), 6.91 (d, 1 H, $J$ = 6 Hz), 7.34 (dd, 1 H, $J$ = 15.5, 6 Hz), 9.52 (s, 1 H). $^{13}C$ NMR ($CDCl_3$) δ194.3 (d), 171.5 (s), 160.0 (d), 146.3 (s), 145.8 (d), 137.8 (s), 136.8 (s), 133.8 (s), 128.3 (d), 126.9 (s), 121.8 (d), 119.5 (d), 97.8 (d), 40.1 (t), 39.7 (t), 34.8 (s), 32.6 (t), 29.5 (t), 28.5 (q), 28.5 (q), 27.7 (t), 24.6 (t), 19.7 (q), 19.4 (t), 16.0 (q).
Mass spectrum, $m/z$ (%), 398 (3), 380 (3), 251 (6), 137 (100).
Mass measurement, $m/z$ = 398.2429, $C_{25}H_{34}O_4$ requires 398.2457.

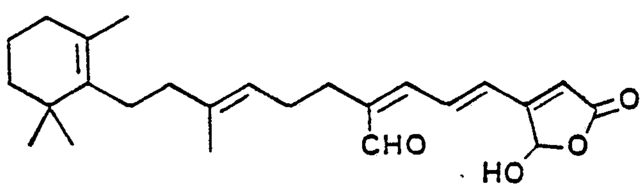

TABLE III
Effect of dehydromanoalide on phorbol-induced inflammation in the mouse ear. (Phorbol myristate acetate, 1.5 µg/ear.)

| Agent | Dose µg/ear | N | Mg. Ear Weight + S.E. |
|---|---|---|---|
| None | — | 13 | 24.60 ± 0.88 |
| Dehydromanoalide | 50 | 8 | 14.13 ± 0.79* |

*Relative Potency*
Equivalent does response to manoalide = 66 µg/ear.
Equivalent does response to dehydromanoalide = 50 µg/ear.
Relative Potency = 1.32.

* Statistically significant decrease (p <0.01) relative to phorbol myristate acetate alone, Student's t-test.

TABLE IV

Effect of manoalide and dehydromanoalide on purified bee venom phospholipase $A_2$.

| | Percent inhibition of control | |
|---|---|---|
| Conc µM | Manoalide | Dehydromanoalide |
| 0.25 | 20.7 | 30.3 |
| 0.50 | 48.0 | 61.8 |
| 0.75 | 73.5 | 51.3 |
| 2.00 | 92.8 | 72.4 |
| 4.00 | 94.5 | 87.3 |

Measurement of phospholipase $A_2$ activity by Radiometer pH stat.
Standard assay conditions: pH 7.4, 41°C, 1.36 mM phosphatidylcholine dipalmitoyl, 2.76 mM triton X—100, and 1.0 mM $Ca^{+2}$.

**Claims**

1. A manoalide derivative of general formula (I):

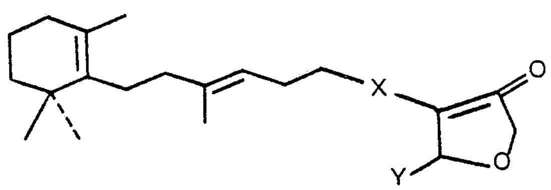

(I)

wherein X is

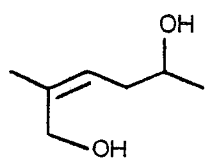

and Y is a hydrogen atom, or X is

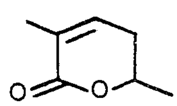

and Y is a hydroxyl or acetoxy group, or X is

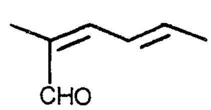

and Y is a hydroxyl group.

2. A compound according to claim 1 which is 3,7-bis(hydroxymethyl)-4-hydroxy-11-methyl-13-(2',6',6'-trimethylcyclohexenyl)-2,6,10-tridecatrienoic acid δ-lactone.

3. A compound according to claim 1 which is manoalide δ-lactone.

4. A compound according to claim 1 which is manoalide δ-lactone acetate.

5. A compound according to claim 1 which is dehydromanoalide

6. A process for the preparation of 3,7-bis(hydroxymethyl)-4-hydroxy-11-methyl-13-(2',6',6'-trimethylcyclohexenyl)-2,6,10-tridecatrienoic acid δ-lactone, which process comprises reacting manoalide with excess sodium borohydride.

7. A process for the preparation of manoalide δ-lactone or manoalide δ-lactone acetate, which process comprises reacting manoalide with Jones' reagent and, if desired, reacting the manoalide δ-lactone thus produced with acetic anhydride to form manoalide δ-lactone acetate.

8. A process for the preparation of dehydromanoalide, which process comprises the acid-catalysed dehydration of manoalide.

9. A pharmaceutical composition comprising, as active ingredient, a compound as claimed in any one of claims 1 to 5 together with a pharmaceutically acceptable additive or excipient.

10. Use of manoalide in the preparation of a pharmaceutical composition for use as an analgesic and/or anti-inflammatory agent.

**Patentansprüche**

1. Manoalidderivat der allgemeinen Formel (I):

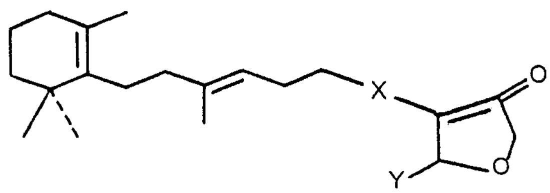

(I)

worin X

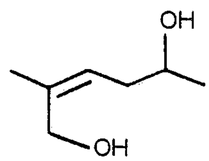

ist und Y ein Wasserstoffatom ist oder X

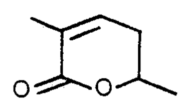

ist und Y eine Hydroxyl- oder Acetoxygruppe ist oder X

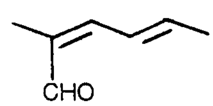

ist und Y eine Hydroxylgruppe ist.

2. Verbindung nach Anspruch 1, welche 3,7-Bis(hydroxymethyl)-4-hydroxy-11-methyl-13-(2',6',6'-trimethylcyclohexenyl)-2,6,10-tridecatriensäure-δ-lacton ist.

3. Verbindung nach Anspruch 1, welche das Manoalid-δ-lacton ist.

4. Verbindung nach Anspruch 1, welche das Manoalid-δ-lactonacetat ist.

5. Verbindung nach Anspruch 1, welche Dehydromanoalid ist.

6. Verfahren zur Herstellung von 3,7-bis(hydroxymethyl)-4-hydroxy-11-methyl-13-(2',6',6'-trimethylcyclohexenyl)-2,6,10-tridecatriensäure-δ-lacton, welches Verfahren die Reaktion von Manoalid mit überschüssigem Natriumborhydrid umfaßt.

7. Verfahren zur Herstellung von Manoalid-δ-lacton oder Manoalid-δ-lactonacetat, welches Verfahren die Reaktion von Manoalid mit Jones' Reagenz und, falls erwünscht, Reaktion des so hergestellten Manoalid-δ-lactons mit Essigsäureanhydrid unter Bildung von Manoalid-δ-lactonacetat umfaßt.

8. Verfahren zur Herstellung von Dehydromanoalid, welches Verfahren die säurekatalysierte Dehydratisierung von Manoalid umfaßt.

9. Pharmazeutische Zusammensetzung, enthaltend als aktiven Bestandteil eine Verbindung, wie in einem der Ansprüche 1 bis 5 beansprucht, zusammen mit einem pharmazeutisch annehmbaren Zusatzstoff oder Excipienten.

10. Verwendung von Manoalid bei der Herstellung einer pharmazeutischen Zusammensetzung zur Verwendung als Analgetikum und/oder entzündungshemmendes Mittel.

**Revendications**

1. Dérivé de manoalide de formule générale (I)

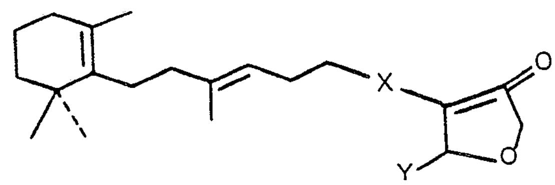

(I)

dans laquelle X est

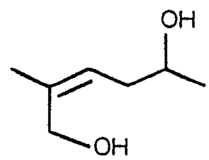

et Y est un atome d'hydrogene, ou bien X est

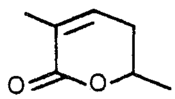

et Y est un groupe hydroxyle ou acétoxy, ou bien X est

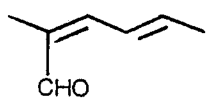

et Y est un groupe hydroxyle.

2. Composé conforme à la revendication 1, qui est la δ-lactone de l'acide, 3,7-bis(hydroxyméthyl)-4-hydroxy-11-méthyl-13-(2',6',6'-triméthylcyclohexènyl)-2,6,10-tridécatriénoïque.

3. Composé conforme à la revendication 1, qui est la δ-lactone de manoalide.

4. Composé conforme à la revendication 1, qui est l'acétate de δ-lactone de manoalide.

5. Composé conforme à la revendication 1, qui est le déhydromanoalide.

6. Procédé pour la préparation de la δ-lactone de l'acide 3,7-bis(hydroxyméthyl)-4-hydroxy-11-méthyl-13-(2',6',6'-triméthylcyclohexènyl)-2,6,10-tridécatriénoïque, lequel procédé comporte la reaction du manoalide avec un excès de borohydrure de sodium.

7. Procédé pour la préparation de la δ-lactone de manoalide ou de l'acétate de δ-lactone de manoalide, lequel procédé comporte la réaction du manoalide avec le réactif de Jones, et si on le désire, la réaction de la δ-lactone de manoalide ainsi produite avec l'anhydride acétique, pour former l'acétate de δ-lactone de manoalide.

8. Procédé pour la preparation du déhydromanoalide, lequel procédé comporte la déshydratation, catalysée par un acide, du manoalide.

9. Composition pharmaceutique comprenant, comme ingrédient actif, un composé tel que revendiqué dans l'une quelconque des revendications 1 à 5, conjointement avec un adjuvant ou un excipient pharmaceutiquement acceptable.

10. Utilisation du manoalide dans la préparation d'une composition pharmaceutique à utiliser comme agent analgésique et/ou anti-inflammatoire.